# EUROPÄISCHE PATENTSCHRIFT

(11) **EP 0 919 282 B1**
(45) Veröffentlichungstag und Bekanntmachung des Hinweises auf die Patenterteilung: **14.08.2002**
(21) Anmeldenummer: 98116794.3
(22) Anmeldetag: 05.09.1998
(51) Int. Cl.: B01L 11/00, A47L 15/50

(54) **Einsatz für einen programmgesteuerten Spülautomaten**
Insert for a programable washing machine
Insert pour une machine à laver programmable

(30) Priorität: 29.11.1997 DE 29721189 U
(43) Veröffentlichungstag der Anmeldung: 02.06.1999
(73) Patentinhaber: Miele & Cie. GmbH & Co., D-33332 Gütersloh (DE)
(72) Erfinder: Liedke, Jürgen, 32130 Enger (DE); Michels, Winfried, Dr., 34414 Warburg (DE)

(56) Entgegenhaltungen:
- DE-A- 2 226 894
- US-A- 3 463 173
- US-A- 5 431 294

## Beschreibung

Der Gegenstand der Erfindung betrifft einen Einsatz für einen programmgesteuerten Spülautomaten zur Aufbereitung von medizinischem Spülgut, wie Pipetten, MIC-Utensilien oder dergl., wobei der Einsatz mit einem an das Spülwasserleitungssystem des Automaten im Spülbehälter andockbaren Spülwasser-Rohrsystem und mit Siebkörben, Halterungen oder dergl. Aufnahmen für die zu reinigenden Spülutensilien ausgebildet ist.

Eine Vorrichtung dieser Galtung ist beispielsweise aus der DE-A-2 226 894 bekannt.

Für das maschinelle Aufbereiten von medizinischem Zubehör in programmgesteuerten Spülautomaten für den Labor- oder Krankenhausbereich ist es bekannt, spülgutspezifische Einsätze, Körbe oder Traggestelle einzusetzen, um die Reinigung einschließlich Desinfektion und Trocknung der Spülutensilien optimal durchführen zu können. Bekannt geworden sind beispielsweise Spezialeinsätze nur für die Aufbereitung von Enghalslaborgläsern, wie Meßkolben oder Pipetten. Auch sind Einsätze nur für Atemschläuche, starre oder flexible Endoskope usw. bekannt.

Die Erfahrung mit den Reinigungs- und Desinfektionsautomaten in der Praxis zeigt, daß oft häufiger als konzeptionell geplant, beim Geräteanwender eine Mischbeladung gewünscht wird, um auch unterschiedliches Spülgut, soweit mit demselben Aufbereitungsprogramm und den spezifischen Behandlungsvorschriften vereinbar, gemeinsam im Spülautomaten zeitsparend aufbereiten zu können. Dies trifft sowohl für Spülutensilien aus dem Krankenhaus- wie auch aus dem Laborbereich zu.

Ausgehend von einem Einsatz der eingangs genannten Art liegt der Erfindung die Aufgabe zugrunde, den Einsatz so auszubilden, daß dem Anwender eine hohe Flexibilität hinsichtlich der Beladung mit unterschiedlichem Spülgut gegeben wird.

Diese Aufgabe wird erfindungsgemäß durch die im Patentanspruch 1 angegebenen Merkmale gelöst.

Vorteilhafte Ausgestaltungen und Weiterbildungen der Erfindung ergeben sich aus den nachfolgenden Unteransprüchen.

Durch den erfindungsgemäßen Einsatz ist dem Anwender eine einfache Möglichkeit gegeben, unterschiedliches Spülgut in einem Einsatz unterzubringen, wobei er nur die entsprechenden Spülgutmodule für die Beladung auszuwählen hat. Die vorteilhafte Trennung bzw. Teilung des Einsatzes in ein als Traggestell ausgebildetes Grundmodul und mit diesem wählbar kombinierbaren Anbaumodulen in Form von Spezialeinsätzen als separate Spülgutmodule für jeweils bestimmtes Spülgut kann der Anwender ohne Probleme eine bestimmte Spülgut-Mischbeladung wählen und diese in einem auf alle eingeräumten Utensilien abgestimmten Reinigungsprogramm des Spülautomaten gemeinsam behandeln. Die Kombinierbarkeit der Module untereinander läßt es zu, für andere Anwendungsbereiche oder auch Maschinenprogramme weitere Module zu schaffen und einzusetzen. So kann das Grundmodul beispielsweise mit austauschbaren Modulen für Inkubationsmaterial, für Anästhesie-Atemschläuche, für MIC-Instrumentarium usw. bestückt werden.

Ein Ausführungsbeispiel der Erfindung ist in den Zeichnungen rein schematisch dargestellt und wird nachfolgend näher beschrieben. Es zeigt
- Figur 1: einen mit separaten Spülgutmodulen in wählbarer Kombination bestückbaren Einsatz eines programmgesteuerten Spülautomaten, in perspektivischer Darstellung,
- Figur 2: ein separates Spülgutmodul zur Aufnahme von Pipetten, in der Voderansicht,
- Figur 3: das Spülgutmodul für Pipetten in der Draufsicht,
- Figur 4: ein separates Spülgutmodul zur Aufnahme von Laborgläsern, in der Vorderansicht,
- Figur 5: das Spülgutmodul für Laborgläser in der Draufsicht,
- Figur 6: ein weiteres Spülgutmodul für Laborgläser, in der Vorderansicht,
- Figur 7: das Spülgutmodul gemäß Fig. 6 in der Draufsicht,
- Figur 8: einen mit Spülgutmodulen bestückten Einsatz, in perspektivischer Darstellung.

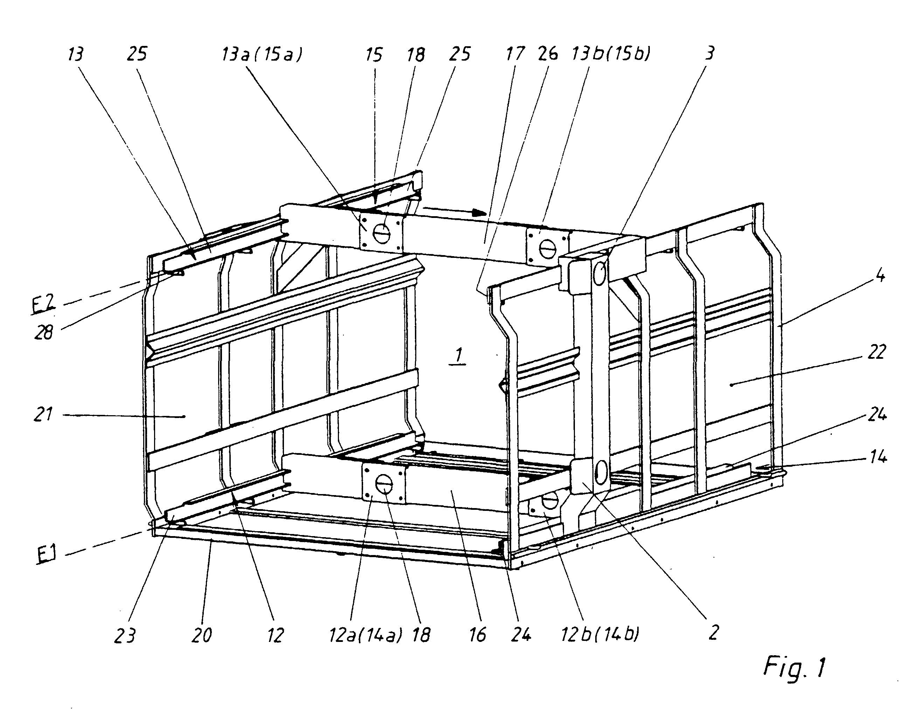

Die Fig 1 zeigt einen Einsatz (1) für einen an sich bekannten programmgesteuerten Spülautomaten (nicht dargestellt), zum Aufbereiten von medizinischem Spülgut, wie Pipetten, MIC-Utensilien oder dergl. Der Einsatz (1) ist mit einem an das Spülwasserleitungssystem des Automaten im Spülbehälter andockbaren Spülwasser-Rohrsystem (2) ausgebildet, welches ggf. auch die Trocknungsluft für das Spülgut heranführt. Die Verbindung mit dem Leitungssystem des Automaten erfolgt beim Einschieben des Einsatzes (1) in das Gerät (sh. Pfeil). Dabei kuppeln am Spülwasser-Rohrsystem (2) des Einsatzes (1) vorgesehene Leitungsadapter (3) die Spülwasserversorgung sowie ggf. die Trocknungsluft an.

Der Einsatz (1) besteht aus einem mit dem Spülwasser-Rohrsystem (2) ausgebildeten Traggestell (4) als Grundmodul und aus mehreren vom Spülwasser-Rohrsystem (2) jeweils separat versorgten und mit dem Grundmodul kombinierbaren Spülgutmodulen (5), Fig. 8, die jeweils bestimmten Spülutensilien (6) zugeordnet werden. So können beispielsweise in einer Charge, wenn das Aufbereitungsprogramm dies vorsieht, gemeinsam Pipetten, MIC-Utensilien und/oder Laborgläser aufbereitet werden.

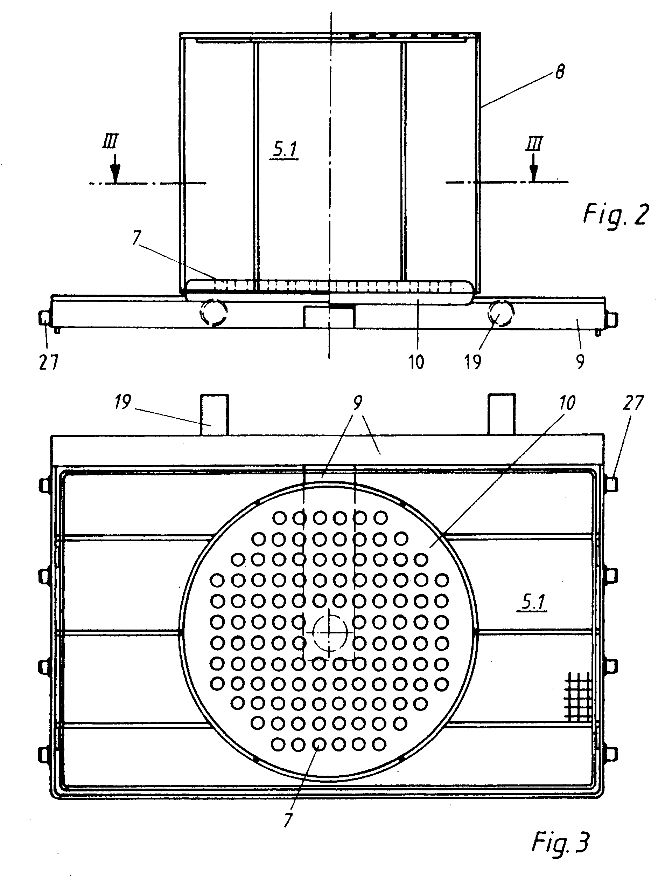

Die unterschiedlichen Spülutensilien (6) werden hierfür in die speziell eingerichteten Spülgutmodule (5) eingeräumt, wobei die Spülgutmodule (5) zur Reinigung der Instrumente, Gläser, Schläuche usw. mit Spritzdüsen (7) an den Spülguthaltem (8), sh. Fig. 2, 3, oder dergl. versehen sind. Die Düsen (7) werden von einem das Spülwasser und ggf. die Trocknungsluft führenden Rohrsystem (9) des Spülgutmoduls (5) versorgt, welches wie beschrieben vom Spülwasser-Rohrsystem (2) des Einsatzes (1) gespeist ist.

In den Fig. 2 bis 7 sind drei separate Spülgutmodule (5.1 bis 5.3) dargestellt, die in wählbarer Kombination mit gegebenenfalls weiteren Modulen eine bestimmte Spülgut-Mischbeladung aufnehmen können. Diese Mischbeladung wird dann in einem auf alle eingeräumten Utensilien (6) abgestimmten Reinigungsprogramm des Spülautomaten aufbereitet, d. h. gespült, gereinigt, ggf. desinfiziert und getrocknet. Es versteht sich, daß anstelle der gezeigten Spülgutmodule (5.1 bis 5.3) je nach Spülgut und Anwendungsprogramm auch andere Module, z. B. für Inkubationsmaterial, Anästhesie-Atemschläuche, MIC-Instrumentarium oder für Spezialinstrumente der Dentaltechnik im Einsatz (1) eingesetzt werden können. Der Einsatz (1) als Grundmodul kann somit in den verschiedenen Anwendungsbereichen genutzt werden. So ist beispielsweise das Spülgutmodul (5.1) gemäß Fig. 2 und 3 für die Aufnahme von Pipetten ausgelegt, die in einem ringförmigen Spülguthalter (8) des Moduls einzeln gehalten und für die Innenreinigung endseitig in Spritzdüsen (7) am Gestellboden (10) des Spülgutmoduls (5.1) eingesteckt sind. Die Spritzdüsen (7) werden vom Rohrsystem (9) mit Spülflüssigkeit versorgt.

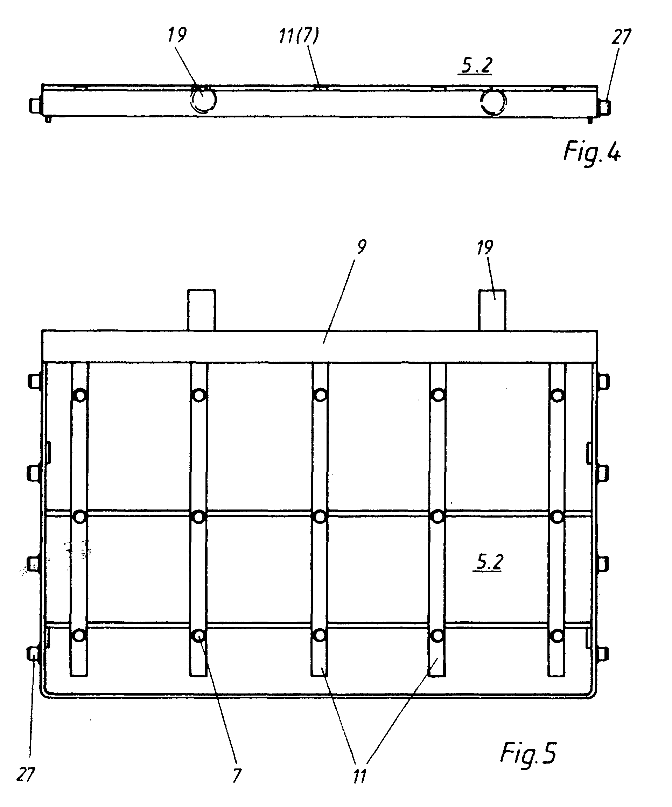

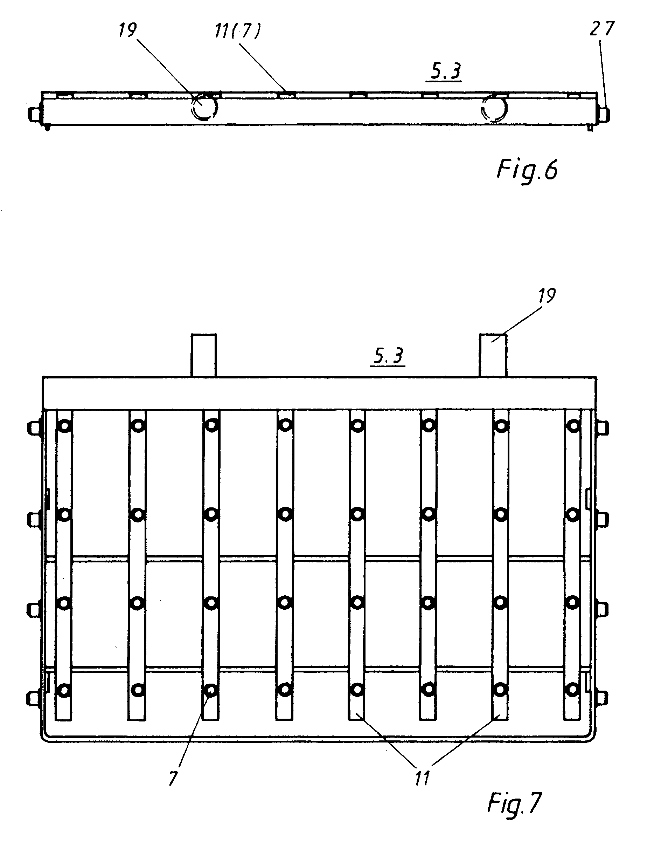

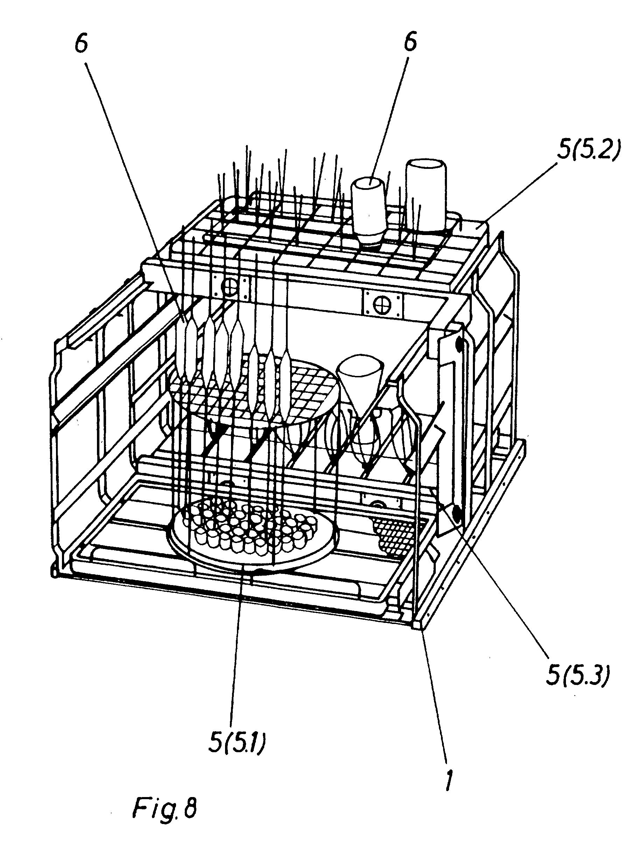

Hingegen können Spülgutmodule (5) für die Aufnahme von verschiedenen Laborgläsern nach Fig. 4 bis 7 ausgebildet sein, wobei diese Spülgutmodule (5.2 und 5.3) mit parallel nebeneinander liegenden Düsenrohren (11) ausgebildet sind. Auf den Düsenrohren (11) sind verteilt angeordnet wiederum die Spritzdüsen (7) ausgebildet. Gegenüber dem Laborglas-Spülgutmodul (5.2) gemäß Fig. 4 und 5 liegen bei dem Laborglas-Spülgutmodul (5.3) nach Fig. 6 und 7 die Düsenrohre (11) dichter nebeneinander. Dieses Spülgutmodul (5.3) kann z. B. für die Aufnahme von Gläsern mit kleinem Außenumfang genutzt werden. Auch ist es möglich, Spülgutmodule (5) zu berücksichtigen bzw. mit einzusetzen, welche nicht spülgutspezifisch gestaltet sind. Dies könnte bspw. für diverse Kleinutensilien nützlich sein.

Die Spülgutmodule (5) sind Einschübe mit gleichen Grundabmessungen, für die im Einsatz (1) in übereinander liegenden Gestellebenen (E1, E2) gleichartige Aufnahmen (12 bis 15) sowie zugeordnete Spülwasseranschlüsse (12a bis 15a und 12b bis 15b), jeweils zwei für eine Aufnahme (12, 13, 14 bzw. 15), vorgesehen sind. Jedes Spülgutmodul (5) besitzt ein moduleigenes auf das einzuräumende Spülgut zugeschnittene Rohrsystem (9), welches an das Spülwasser-Rohrsystem (2) des Traggestells (4) beim Einsetzen in das Grundmodul selbsttätig andockt. Damit die Spülflüssigkeit auch auf alle eingeschobenen Spülguteinsätze (5) geleitet werden kann, ist das Spülwasser-Rohrsystem (2) des Einsatzes (1) über zwei Rohrverbinder (16, 17) zur Herstellung der Spülwasseranschlüsse (12a bis 15a und 12b bis 15b) in die einzelnen Gestellebenen (E1, E2) für die Spülgutmodule (5) verzweigt.

Die Spülwasseranschlüsse (12a bis 15a und 12b bis 15b) der Rohrverbinder (16, 17) sind jeweils mit einem Rückschlagventil (18) versehen, welche bei nicht eingeschobenem Spülgutmodul (5) den Wasseraustritt absperren. Für die Verbindung der Spülgutmodule (5) mit den Rohrverbindern (16, 17) sind die Rohrsysteme (9) der Spülgutmodule (5) jeweils mit zwei Anschlußstutzen (19) versehen, welche in die Spülwasseranschlüsse (12a bis 15a und 12b bis 15b) eintauchen und dabei selbsttätig die Rückschlagventile (18) öffnen. Die Ventile können als Klappenventile ausgebildet sein.

Der Einsatz (1) als Grundmodul ist annähernd U-förmig mit einem Gestellboden (20) und zwei senkrecht dazu mit dem Boden verbundenen Gestellwänden (21, 22) ausgebildet. Die Gestellwände (21, 22) sind mittig jeweils oben und im Bodenbereich von einem Rohrverbinder (16 bzw. 17) des Spülwasser-Rohrsystems (2) überbrückt, wobei beiderseits der Rohrverbinder (16, 17) die Aufnahmen (12 bis 15) für die Spülgutmodule (5) in den Gestellwänden (21, 22) des Traggestells (4) ausgebildet sind. Die Aufnahmen (12 bis 15) für die Spülgutmodule (5) sind als in Längsrichtung der Gestellwände (21, 22) verlaufende Führungsschienen (23 bis 26) ausgebildet, wobei pro Gestellebene (E1 bzw. E2) jeweils zwei Führungsschienen (23, 24 bzw. 25, 26) einem Spülgutmodul (5) zugeordnet sind.

Das korbähnlich gestaltete Traggestell (4) nimmt zwischen seinen beiden Gestellwänden (21, 22) in den von den Rohrverbindern (16, 17) getrennten beiden Seitenräumen die separaten Spülgutmodule (5) auf. Die Spülgutmodule (5) besitzen für ein leichtes Einsetzen und Herausziehen in bzw. aus dem Einsatz (1) seitlich am Grundrahmen Laufräder (27) oder Gleitstücke. Die Sicherung der im Einsatz (1) eingesetzten Spülgutmodule (5) erfolgt durch Feststelleinrichtungen, die in einfachster Form bspw. quer zur Einschubrichtung hinter einem Spülgutmodul (5) am Traggestell (4) angebrachte Sicherungsstege (28) sein können.

Die Fig. 8 zeigt einen mit Spülgutmodulen (5) bestückten Einsatz (1) in perspektivischer Darstellung, wobei das Spülgutmodul (5.1) für Pipetten gemäß Fig. 3 und 4 in der unteren Gestellebene (E1) des Einsatzes (1) eingeschoben ist. Aufgrund der hier sehr langen Spülutensilien (Pipetten 6) ist eine Belegung der über den Pipetten im Einsatz (1) liegenden Gestellebene (E2) mit einem weiteren Spülgutmodul (5) nicht mehr vorgesehen. Im gegenüberliegenden Seitenraum (28) des Grundmoduls sind beide Gestellebenen (E1, E2) jedoch frei für die beiden Laborglas-Spülguteinsätze (5.2 und 5.3) gemäß den Fig. 4 bis 7. Die Versorgung der Module mit Spülflüssigkeit und ggf. Trocknungsluft kann über die entsprechend freigeschalteten Rückschlagventile (18) der Rohrverbinder (16, 17) des Einsatzes (1) erfolgen. Für die Außenreinigung der Spülutensilien (6) können an den Spülgutmodulen (5), am Einsatz (1) und/oder im Spülraum des Spülautomaten entsprechende Sprühvorrichtungen oder Düsen vorgesehen werden.

## Patentansprüche

1. Einsatz für einen programmgesteuerten Spülautomaten zur Aufbereitung von medizinischem Spülgut, wie Pipetten, MIC-Utensilien oder dergl., wobei der Einsatz mit einem an das Spülwasserleitungssystem des Automaten im Spülbehälter andockbaren Spülwasser-Rohrsystem und mit Siebkörben, Halterungen oder dergl. Aufnahmen für die zu reinigenden Spülutensilien ausgebildet ist,
**dadurch gekennzeichnet,**
**daß** der Einsatz (1) aus einem mit dem Spülwasser-Rohrsystem (2) ausgebildeten Traggestell (4) als Grundmodul und aus mehreren vom Spülwasser-Rohrsystem (2) jeweils separat versorgten und mit dem Grundmodul kombinierbaren Spülgutmodulen (5; 5.1 bis 5.3) jeweils für bestimmte Spülutensilien (6) besteht.

2. Einsatz nach Anspruch 1,
**dadurch gekennzeichnet,**
**daß** die Spülgutmodule (5; 5.1 bis 5.3) als Einschübe mit gleichen Grundabmessungen ausgebildet sind und daß im Grundmodul in übereinander liegenden Gestellebenen (E1, E2) gleichartige Aufnahmen (12 bis 15) sowie zugeordnete Spülwasseranschlüsse (12a bis 15a und 12b bis 15b) für die Spülgutmodule (5; 5.1 bis 5.3) vorgesehen sind.

3. Einsatz nach Anspruch 1 und 2,
**dadurch gekennzeichnet,**
**daß** jedes Spülgutmodul (5; 5.1 bis 5.3) mit einem an das Spülwasser-Rohrsystem (2) des Einsatzes (1) andockbaren eigenen Rohrsystem (9) ausgebildet ist.

4. Einsatz nach einem oder mehreren der Ansprüche 1 bis 3,
**dadurch gekennzeichnet,**
**daß** das Spülwasser-Rohrsystem (2) des Traggestells (4) über Rohrverbinder (16, 17) in die Gestellebenen (E1, E2) für die Spülgutmodule (5; 5.1 bis 5.3) verzweigt ist.

5. Einsatz nach einem oder mehreren der Ansprüche 1 bis 4,
**dadurch gekennzeichnet,**
**daß** die Rohrverbinder (16, 17) des Spülwasser-Rohrsystems (2) für die andockbaren Spülgutmodule (5; 5.1 bis 5.3) mit Rückschlagventilen (18) ausgebildet sind, die bei unbelegtem Anschluß einen Wasseraustritt verhindern.

6. Einsatz nach einem oder mehreren der Ansprüche 1 bis 5,
**dadurch gekennzeichnet,**
**daß** die Rohrsysteme (9) der Spülgutmodule (5; 5.1 bis 5.3) mit in die Rohrverbinder (16, 17) des Grundmoduls einsteckbaren die Rückschlagventile (18) öffnenden Anschlußstutzen (19) versehen sind.

7. Einsatz nach einem oder mehreren der Ansprüche 1 bis 6,
**dadurch gekennzeichnet,**
**daß** an den Rohrsystemen (9) der Spülgutmodule (5; 5.1 bis 5.3) Spritzdüsen (7) für eine Innen- und/oder Außenreinigung der Spülutensilien (6) ausgebildet sind.

8. Einsatz nach einem oder mehreren der Ansprüche 1 bis 7,
**dadurch gekennzeichnet,**
**daß** zur Sicherung der Spülgutmodule (5; 5.1 bis 5.3) im Traggestell (4) Feststelleinrichtungen vorgesehen sind.

9. Einsatz nach einem oder mehreren der Ansprüche 1 bis 8,
**dadurch gekennzeichnet,**
**daß** der Einsatz (1) als Grundmodul annähernd U-förmig mit einem Gestellboden (20) und zwei senkrecht dazu mit dem Boden verbundenen Gestellwänden (21, 22) ausgebildet ist, welche mittig jeweils oben und im Bodenbereich von einem Rohrverbinder (16, 17) des Spülwasser-Rohrsystems (2) überbrückt sind, wobei beiderseits der Rohrverbinder (16, 17) die Aufnahmen (12 bis 15) für die Spülgutmodule (5; 5.1 bis 5.3) in den Gestellwänden (21, 22) ausgebildet sind.

10. Einsatz nach einem oder mehreren der Ansprüche 1 bis 9,
**dadurch gekennzeichnet,**
**daß** die Aufnahmen (12 bis 15) für die Spülgutmodul (5; 5.1 bis 5.3) in Längsrichtung der Gestellwände (21, 22) verlaufende Führungsschienen (23 bis 26) sind, und daß pro Gestellebene (E1 bzw. E2) jeweils zwei Führungsschienen (23, 24 bzw. 25, 26) einem Spülgutmodul (5) zugeordnet sind.

## Claims

1. Insert for a program-controlled automatic washing machine for processing medical items to be washed, such as pipettes, MIC instruments or the like, the insert being provided with a system of water tubes, which system is positionable on the system of water pipes of the automatic machine in the washing chamber, and being provided with perforated baskets, holders or similar receiving means for the instruments to be cleaned, **characterised in that** the insert (1) comprises a carrying rack (4) as the basic module, which is provided with the system (2) of water tubes, and a plurality of modules of items to be washed (5; 5.1 to 5.3), which are supplied separately by the system (2) of water tubes and can be combined with the basic module, each module of items to be washed being for specific instruments to be washed (6).

2. Insert according to claim 1, **characterised in that** the modules of items to be washed (5; 5.1 to 5.3) are configured as slide-in units with identical basic dimensions, and **in that** similar receiving means (12 to 15) as well as associated rinsing water connections (12a to 15a and 12b to 15b) for the modules of items to be washed (5; 5.1 to 5.3) are provided in the basic module in rack planes (E1, E2) lying one above the other.

3. Insert according to claims 1 and 2, **characterised in that** each module of items to be washed (5; 5.1 to 5.3) is provided with its own tube system (9), which is positionable on the system (2) of water tubes of the insert (1).

4. Insert according to one or more of claims 1 to 3, **characterised in that** the system (2) of water tubes of the carrying rack (4) is branched into the rack planes (E1, E2) for the modules of items to be washed (5; 5.1 to 5.3) via tube connectors (16, 17).

5. Insert according to one or more of claims 1 to 4, **characterised in that** the tube connectors (16, 17) of the system (2) of water tubes for the positionable modules of items to be washed (5; 5.1 to 5.3) are provided with non-return valves (18) which prevent water from escaping if the connection is not established.

6. Insert according to one or more of claims 1 to 5, **characterised in that** the tube systems (9) of the modules of items to be washed (5; 5.1 to 5.3) are provided with connection-pieces (19) which are insertable into the tube connectors (16, 17) of the basic module and open the non-return valves (18).

7. Insert according to one or more of claims 1 to 6, **characterised in that** spray nozzles (7) for cleaning the instruments to be washed (6) internally and/or externally are provided on the tube systems (9) of the modules of items to be washed (5; 5.1 to 5.3).

8. Insert according to one or more of claims 1 to 7, **characterised in that**, to secure the modules of items to be washed (5; 5.1 to 5.3), locking means are provided in the carrying rack (4).

9. Insert according to one or more of claims 1 to 8, **characterised in that** the insert (1), as the basic module, has an approximately U-shaped configuration with a rack base (20) and two rack walls (21, 22), which are connected to the base perpendicularly to said base and are each spanned centrally, at the upper end and in the base region, by a tube connector (16, 17) of the system (2) of water tubes, the receiving means (12 to 15) for the modules of items to be washed (5; 5.1 to 5.3) being provided in the rack walls (21, 22) on each side of the tube connectors (16, 17).

10. Insert according to one or more of claims 1 to 9, **characterised in that** the receiving means (12 to 15) for the modules of items to be washed (5; 5.1 to 5.3) are guide rails (23 to 26) extending in the longitudinal direction of the rack walls (21, 22), and **in that** each module of items to be washed (5) has associated therewith two guide rails (23, 24 or respectively 25, 26) per rack plane (E1 or E2 respectively).

## Revendications

1. Insert pour une machine à laver commandée par programme destiné au traitement d'ustensiles médicaux tels que des pipettes, des instruments utilisés en chirurgie peu invasive ou analogue, l'insert comportant une tuyauterie pour l'eau pouvant être raccordée à la conduite d'arrivée d'eau de la machine dans la cuve de lavage, ainsi que des filtres, des dispositifs de fixation ou autres réceptacles pour les ustensiles à nettoyer, **caractérisé en ce que** l'insert (1) est composé d'un bâti (4) équipé de la tuyauterie pour l'eau (2) qui constitue le module de base et de plusieurs modules (5 ; 5.1 à 5.3) destinés chacun à un certain type d'ustensiles (6), qui sont alimentés séparément par la tuyauterie pour l'eau (2) et qui peuvent être combinés au module de base.

2. Insert selon la revendication 1, **caractérisé en ce que** les modules à ustensiles (5 ; 5.1 à 5.3) sont réalisés sous forme d'éléments enfichables ayant tous les mêmes dimensions de base et **en ce que**, dans le module de base, à des niveaux superposés (E1, E2) du bâti, sont prévus des réceptacles similaires (12 à 15), ainsi que des raccords d'eau (12a à 15a et 12b à 15b) pour les modules (5 ; 5.1 à 5.3) destinés aux différents ustensiles.

3. Insert selon les revendications 1 et 2, **caractérisé en ce que** chaque module à ustensiles (5 ; 5.1 à 5.3) est doté d'une tuyauterie (9) propre pouvant être raccordée à la tuyauterie pour l'eau (2).

4. Insert selon l'une ou plusieurs des revendications 1 à 3, **caractérisé en ce que** la tuyauterie pour l'eau (2) du bâti (4) se ramifie par le biais de raccords de tuyaux (16, 17) vers les différents niveaux (E1, E2) du bâti pour les modules à ustensiles (5 ; 5.1 à 5.3).

5. Insert selon l'une ou plusieurs des revendications 1 à 4, **caractérisé en ce que** les raccords de tuyaux (16, 17) de la tuyauterie pour l'eau (2) qui sont destinés aux modules pouvant être raccordés (5 ; 5.1 à 5.3) sont équipés de clapets anti-retour (18) afin d'empêcher l'eau de s'écouler d'un raccord qui n'est pas utilisé.

6. Insert selon l'une ou plusieurs des revendications 1 à 5, **caractérisé en ce que** les tuyauteries (9) des modules (5 ; 5.1 à 5.3) sont munies d'embouts (19) pouvant être enfoncés dans les raccords (16, 17) du module de base et ouvrant les clapets anti-retour (18).

7. Insert selon l'une ou plusieurs des revendications 1 à 6, **caractérisé en ce que** des buses d'aspersion (7) pour nettoyer les ustensiles (6) à l'intérieur et/ou à l'extérieur sont réalisées sur les tuyauteries (9) des modules à ustensiles (5 ; 5.1 à 5.3).

8. Insert selon l'une ou plusieurs des revendications 1 à 7, **caractérisé en ce que** des dispositifs de fixation sont prévus dans le bâti (4) pour assurer les modules à ustensiles (5; 5.1 à 5.3).

9. Insert selon l'une ou plusieurs des revendications 1 à 8, **caractérisé en ce que** l'insert (1) en tant que module de base est réalisé approximativement en forme de U avec un fond (20) et deux parois (21, 22) assemblées perpendiculairement avec ce dernier, qui sont reliées entre elles au milieu, en haut et au niveau du fond, par un raccord de tuyau (16, 17) de la tuyauterie pour l'eau (2), sachant que de part et d'autre des raccords de tuyaux (16, 17), les réceptacles (12 à 15) pour les modules à ustensiles (5 ; 5.1 à 5.3) sont réalisés dans les parois (21, 22) du bâti.

10. Insert selon l'une ou plusieurs des revendications 1 à 9, **caractérisé en ce que** les réceptacles (12 à 15) pour les modules à ustensiles (5 ; 5.1 à 5.3) sont des rails de guidage (23 à 26) s'étendant dans la direction longitudinale des parois (21, 22) du bâti, et **en ce qu'**à chaque niveau du bâti (respectivement E1 et E2), deux rails de guidage (respectivement 23, 24 et 25, 26) sont associés à un module à ustensiles (5).
